# EUROPEAN PATENT APPLICATION

(11) **EP 4 477 743 A1**
(43) Date of publication of application: **18.12.2024**
(21) Application number: 23749882.9
(22) Date of filing: 06.02.2023
(51) Int. Cl.: C12N 5/02, C12N 5/0735, C12N 5/10

(54) **DIFFERENTIATION INDUCTION CONTROL AGENT AND DIFFERENTIATION INDUCTION STABILIZING AGENT FOR PLURIPOTENT STEM CELLS**

(30) Priority: 07.02.2022 JP 2022017548
(71) Applicant: OSAKA UNIVERSITY, Suita-shi Osaka 565-0871 (JP)
(72) Inventor: KINOOKA, Masahiro, Suita-shi, Osaka 565-0871 (JP); KIM, Meehae, Suita-shi, Osaka 565-0871 (JP); FUJINAGA, Yukako, Suita-shi, Osaka 565-0871 (JP)
(74) Representative: Hoffmann Eitle
(86) International application number: PCT/JP2023/003689
(87) International publication number: WO 2023/149566

(57) **Abstract**

The present invention provides an agent for directional control of differentiation induction of a pluripotent stem cell, comprising an E-cadherin inhibitor.

## Description

### [Technical Field]

The present invention relates to a control agent of the induction of differentiation of pluripotent stem cells and a stabilizing agent of the differentiation induction of pluripotent stem cells.

### [Background Art]

In the stem cell industry, including regenerative medicine, it is necessary to produce differentiated cells of interest in vitro in large amounts and in a uniform and homogeneous manner. As a cell source in this case, pluripotent stem cells may be used. In this cell production (differentiation induction) process, it is necessary to induce differentiation of pluripotent stem cells into desired cells, mature the cells, and maintain the desired differentiation state as uniformly as possible.

However, in the process of inducing differentiation from pluripotent stem cells into the desired cells in vitro, undesired cells may be generated. In addition, the speed of differentiation induction may vary between individual cells, and uniformly differentiated cells may not be obtained. Furthermore, even if differentiation induction is performed under the same conditions, the quality of the cells obtained may vary depending on the technique.

When induction of differentiation from pluripotent stem cells into the desired cells in vitro is attempted, it is sometimes very difficult to obtain a population of uniformly and homogeneously differentiated cells. While many methods for differentiation induction and many methods for increasing the induction efficiency have been reported, there is still no useful universal method for obtaining a uniform cell population, which forms a major obstacle to the production of uniform and high-quality cellular medicines.

In the differentiation induction process of pluripotent stem cells, attempts have been made to increase the production amount of a certain, desired differentiated cell or improve the efficiency of differentiation induction by using an E-cadherin inhibitor (Patent Literatures 1 and 2).

Patent Literature 1 discloses a method of acting an E-cadherin inhibitor on human embryonic stem (ES) cells and improving the efficiency of differentiation into definitive endoderm cells by using TGFβ, thereby increasing the production amount of the cells. In addition, Patent Literature 2 discloses that, in a method of inducing differentiation of ES or induced pluripotent stem (iPS) cells, E-cadherin inhibitors delay differentiation into most cell lineages but do not delay differentiation into neural progenitor cells, and as a result, the E-cadherin inhibitors contribute to the induction of differentiation into neural progenitor cells.

On the other hand, the present inventors have reported that, in the maintenance culture process of pluripotent stem cells, hemagglutinin or a variant thereof, which is an E-cadherin inhibitor, was used to maintain an undifferentiated state, thereby enabling the mass culture of the pluripotent stem cells (Patent Literatures 3 to 5).

Patent Literatures 3 and 4 disclose methods for removing cells that have deviated from the undifferentiated state and have occurred or may occur during the culture of pluripotent stem cells, by using hemagglutinin or a variant thereof. Patent Literature 4 discloses a method for dividing cell aggregates in suspension culture of iPS cells into small clumps by using hemagglutinin. Furthermore, Patent Literature 5 discloses a method for enabling mass culture of pluripotent stem cells by using hemagglutinin or a variant thereof.

However, there are no reports that E-cadherin inhibitors contribute to differentiation induction of pluripotent stem cells, other than Patent Literatures 1 and 2 showing differentiation induction into definitive endoderm cells or neural precursor cells. In addition, the inventions described in Patent Literatures 3 to 5 are techniques relating to the maintenance and expansion culture of pluripotent stem cells, and are not techniques relating to differentiation induction.

### [Citation List]

### [Patent Literature]

[Patent Literature 1]
   WO2009/041984
[Patent Literature 2]
   WO2014/072720
[Patent Literature 3]
   WO2014/104207
[Patent Literature 4]
   WO2015/199243
[Patent Literature 5]
   WO2018/117110

### [Summary of Invention]

### [Technical Problem]

It is an object of the present invention to provide a means of controlling the differentiation of pluripotent stem cells that makes it possible to provide any desired differentiated cells as a uniform and homogeneous cell population from pluripotent stem cells while suppressing the generation of undesired cells. Also, an object of the present invention is to provide a method for stably inducing differentiation of pluripotent stem cells into the desired cells, by using the differentiation controlling means.

### [Solution to Problem]

The present inventors have previously been involved in developing technologies for removing "cells deviated from the undifferentiated state" that arise during the cultivation of pluripotent stem cells, as well as technologies for cultivating "cells maintaining the undifferentiated state" with the aim of large-scale production of pluripotent stem cells. The technology in question is hypothesized to function through a mechanism wherein hemagglutinin, an inhibitor of the cell adhesion molecule E-cadherin, specifically binds to E-cadherin, thereby inhibiting intercellular adhesion and consequently relaxing intercellular adhesion (WO2014/104207, WO2015/199243 and WO2018/117110). This relaxation of intercellular adhesion is considered to create a more uniform and homogeneous cultivation environment for each cell within the pluripotent stem cell colony, potentially resulting in an environment less conducive to the emergence of deviated cells and more favorable for cell proliferation.

The present inventors developed the above-mentioned finding relating to the maintenance of an undifferentiated state of pluripotent stem cells. When conventional differentiation induction methods are used in inducing differentiation of pluripotent stem cells into the desired cells in vitro, variations in cell growth and differentiation state occur, resulting in the occurrence of undesired cells. The present inventors considered that this might be because the degree of intercellular adhesion varies depending on the position within the aggregate during the process of proliferation and formation of aggregates (colonies) of the pluripotent stem cells. More specifically, the present inventors made a hypothesis that the cells are not so crowded at the periphery of the colony or aggregate, and the intercellular adhesion is not strong, but inside the colony or aggregate, the cells are crowded and strongly adhere to each other, and as a result, the differentiation induction environment for the pluripotent stem cells that constitute the colony or aggregate is different, resulting in the occurrence of cells that have deviated from the desired differentiation state.

The present inventors thus attempted to make uniform the intercellular adhesive force in the aggregates by adding hemagglutinin capable of inhibiting E-cadherin, which is a protein that controls intercellular adhesion, to a differentiation induction system for pluripotent stem cells. As a result, it was unexpectedly discovered that by varying the concentration of hemagglutinin, it is possible to control the direction of differentiation of pluripotent stem cells towards any of the three germ layer lineages, i.e., to direct differentiation towards the desired lineage of any of the three germ layer lineages. Furthermore, it was found that administering hemagglutinin could enhance the efficiency of differentiation induction. These results strongly suggest that E-cadherin inhibition not only makes uniform the intercellular adhesion force within the cell aggregates but also alters the intercellular adhesion force according to the inhibition activity, leading to qualitative changes in the intracellular environment of pluripotent stem cells and potentially altering the directionality of differentiation induction.

Based on these findings, the present inventors further conducted intensive studies and completed the present invention.

That is, the present invention provides the following.

### (Item 1)

An agent for directional control of differentiation induction of a pluripotent stem cell, comprising an E-cadherin inhibitor.

### (Item 2)

The agent of Item 1, wherein the pluripotent stem cell is an embryonic stem (ES) cell or an induced pluripotent stem (iPS) cell.

### (Item 3)

The agent of Item 1 or 2, wherein the directional control of differentiation induction of pluripotent stem cell is a change in the differentiation efficiency of pluripotent stem cell into any of the three germ lineages.

### (Item 4)

The agent of Item 1 or 2, wherein the directional control of differentiation induction of pluripotent stem cell is a reduction or removal of cells that deviate from the desired differentiated cell.

### (Item 5)

The agent of any one of Items 1 to 4, wherein the E-cadherin inhibitor is hemagglutinin.

### (Item 6)

A method for controlling the directionality of differentiation induction of a pluripotent stem cell, comprising a step of culturing a pluripotent stem cell in a medium comprising an E-cadherin inhibitor.

### (Item 7)

The method of Item 6, wherein the culture is performed in the presence of a differentiation inducer.

### (Item 8)

The method of Item 6 or 7, wherein, in the step of culturing a pluripotent stem cell in a medium comprising an E-cadherin inhibitor, a concentration of the inhibitor is adjusted.

### (Item 9)

The method of any one of Items 6 to 8, wherein the directional control of differentiation induction of pluripotent stem cell is a change in the differentiation efficiency of pluripotent stem cell into any of the three germ lineages.

### (Item 10)

The method of any one of Items 6 to 9, wherein the E-cadherin inhibitor is hemagglutinin.

### (Item 11)

An agent for stabilizing differentiation induction of a pluripotent stem cell, comprising an E-cadherin inhibitor.

### (Item 12)

The agent of Item 11, wherein the stabilization of differentiation induction of pluripotent stem cell is an improvement in the differentiation efficiency of pluripotent stem cell into any of the three germ lineages.

### (Item 13)

The agent of Item 11, wherein the stabilization of differentiation induction of pluripotent stem cell is a reduction or removal of cells that deviate from the desired differentiated cell.

### (Item 14)

The agent of any one of Items 11 to 13, wherein the E-cadherin inhibitor is hemagglutinin.

### (Item 15)

A method for producing a desired differentiated cell from a pluripotent stem cell, comprising the following steps:
(1) a step of forming an aggregate of pluripotent stem cells, and
(2) a step of culturing the aggregates obtained in step (1) in a medium comprising an E-cadherin inhibitor, thereby to stabilize the differentiation induction into the desired differentiated cell.

### (Item 16)

The method of Item 15, wherein the culture is performed in the presence of a differentiation inducer.

### (Item 17)

The method of Item 15 or 16, wherein the pluripotent stem cell is an ES cell or an iPS cell.

### (Item 18)

The method of any one of Items 15 to 17, wherein the E-cadherin inhibitor is hemagglutinin.

### [Advantageous Effects of Invention]

According to the present invention, it is possible to control the directionality of differentiation induction of pluripotent stem cells. Furthermore, the present invention allows for the stabilization of differentiation induction from pluripotent stem cells to the desired cells. Specifically, by varying the concentration of E-cadherin inhibitors (preferably, hemagglutinin), the environment for cell differentiation induction can be adjusted, thereby enabling the direction of differentiation towards any of cells of the three germ layer lineages. Moreover, by using E-cadherin inhibitors (preferably, hemagglutinin) to create a suitable and uniform intercellular adhesion environment for the desired differentiated cells throughout the entire culture system, it is possible to significantly improve the efficiency of differentiation induction towards the desired differentiated cells.

### [Brief Description of Drawings]

[Fig. 1]
   The figure shows microscopic images taken on the 40th day of culturing human iPS cells in the absence of a differentiation inducer that promotes differentiation induction of retinal pigment epithelial (RPE) cells, using known basic RPE cell differentiation induction medium, which either does not contain hemagglutinin or contains hemagglutinin at various concentrations, and RPE cell maintenance medium, showing the differentiation into (left) neuroretinal progenitor cell-like aggregates, (center) hepatocyte-like cells, and (right) muscular cell-like cells. A portion of the upper panel is enlarged in the lower panel.
[Fig. 2]
   The figure shows the results of comparing the expression levels of marker genes of the desired germ line when human iPS cells were cultured in medium containing various concentrations of hemagglutinin for 24 hours and then further cultured in medium for inducing differentiation into each of the three germ layers. The vertical axis indicates the relative expression of each gene with the expression level of the gene when cultured in hemagglutinin-free medium set as 1. *:p<0.05, **:p<0.1
[Fig. 3]
   The figure shows the results of comparing the expression levels of marker genes in cardiomyocytes when human iPS cells were cultured in medium containing various concentrations of hemagglutinin for 24 hours and then further cultured in medium for inducing differentiation into cardiomyocytes. The vertical axis indicates the relative expression of each gene with the expression level of the gene when cultured in hemagglutinin-free medium set as 1. *:p<0.05, **:p<0.1
[Fig. 4]
   The figure is a graph (box-and-whisker diagram) quantifying the percentage of cTnT-positive cells and their dispersion trend when human iPS cells were cultured in the medium either without hemagglutinin or containing 0.5 nM of hemagglutinin to induce differentiation into cardiomyocytes. In the graph, the squares indicate the interquartile range (IQR), and the horizontal line within the square indicates the median. The bars indicate the range from the minimum value within the first quartile -1.5 IQR to the maximum value within the third quartile +1.5 IQR. Data outside this range were considered outliers.
[Fig. 5]
   The figure shows the comparison of expression levels of marker genes of various cells when human iPS cells were cultured in medium containing various concentrations of hemagglutinin for 3 days and then further cultured in medium for inducing differentiation into hepatocytes. (A) Comparison of expression levels of pluripotency marker genes, endoderm marker genes, and common hepatic marker genes at 3 days after the start of hepatocyte induction (after endoderm induction treatment). (B) Comparison of expression levels of hepatic progenitor marker gene, immature hepatocyte marker gene, common hepatic marker gene, and mature hepatocyte marker genes at 17 days after the start of hepatocyte induction (after hepatocyte maturation treatment). The vertical axis indicates the relative expression levels of each gene with the expression level of the gene when cultured in hemagglutinin-free medium set as 1. *:p<0.05, **:p<0.1

### [Description of Embodiments]

The present invention is based, at least in part, on the previous study results of the present inventors, showing that cells that have deviated from an undifferentiated state occur within a colony in the maintenance and expansion culture of pluripotent stem cells because the strength of intercellular adhesion force caused by E-cadherin varies within the colony, which in turn causes ununiform sensitivity of each pluripotent stem cell to the medium environment that supports the maintenance of the undifferentiated state. In other words, based on the findings obtained by the maintenance and expansion technology of pluripotent stem cells, the present inventors took note of the inhibition of intercellular adhesion by E-cadherin inhibitors, and assumed that this inhibition also contributes to the "uniformization and homogenization" of the intracellular environment during the differentiation induction of pluripotent stem cells, and, as a result, affords qualitative and quantitative improvements also in the desired cells obtained in the differentiation induction system from pluripotent stem cells. This hypothesis may explain the apparently contradictory phenomenon that E-cadherin not only maintain the undifferentiated state of pluripotent stem cells, but also contribute to differentiation induction into certain differentiated cells.

Remarkably, it has also been revealed that the degree of inhibition of intercellular adhesion by hemagglutinin, an E-cadherin inhibitor (specifically, variations in the concentration of hemagglutinin in the medium, treatment time with hemagglutinin, and timing of hemagglutinin treatment, etc.), affects the phenotypic differences of the resulting differentiated cells. This is likely because differences in intercellular adhesion strength impact the "directionality of differentiation induction" during the early stages of differentiation induction. This suggests that by appropriately adjusting the inhibitory activity of E-cadherin (i.e., the concentration of the E-cadherin inhibitor added), it is possible to efficiently (in a more uniform and homogeneous manner) induce differentiation towards any desired cell type.

The contents of the present invention are described in detail below.

### 1. The control agent of the present invention

The present invention provides an agent for directional control of the differentiation induction of pluripotent stem cells, containing an E-cadherin inhibitor. In the following, it is at times referred to as "the control agent of the present invention".

### [Pluripotent stem cell]

In the present specification, the pluripotent stem cell is not particularly limited as long as it has the "self-renewal ability" to proliferate while maintaining an undifferentiated state and the "differentiation pluripotency" to differentiate into all three germ lineages. Examples thereof include induced pluripotent stem cells (iPS cells) and embryonic stem cells (ES cells). In addition, examples of the pluripotent stem cell include pluripotent stem cells derived from mammals. Pluripotent stem cells derived from mammals are not particularly limited, and examples thereof include human pluripotent stem cells. Human pluripotent stem cells are not particularly limited, and examples thereof include human iPS cells and human ES cells.

In the context of the control agent of the present invention, "differentiation induction" in the "directionality control of differentiation induction of pluripotent stem cell" refers to placing pluripotent stem cells in an environment that does not support its maintenance of the undifferentiated state. This includes not only placing the pluripotent stem cells in the presence of differentiation inducers that support differentiation into a specific desired cell type but also placing them in the absence of substances that support the maintenance of the undifferentiated state (e.g., bFGF, SCF, LIF). Specifically, this encompasses various processes such as the formation of embryoid bodies from pluripotent stem cells, differentiation into any of the three germ layers (e.g., differentiation into ectoderm from pluripotent stem cells, differentiation into mesoderm from pluripotent stem cells, or differentiation into endoderm from pluripotent stem cells) from pluripotent stem cells, and differentiation into ectodermal, mesodermal, or endodermal lineage cells from pluripotent stem cells.

In this context, ectodermal lineage cells are not particularly limited and may include, for example, neural cells and epidermal cells. Specifically, retinal pigment epithelial cells are mentioned as examples.

Mesodermal lineage cells are also not particularly limited and may include, for example, hematopoietic cells (including blood-lineage cells), vascular system cells (vascular endothelial cells etc.), cardiomyocytes (e.g., atrial myocytes, ventricular myocytes etc.), osteocytes, chondrocytes, tendon cells, adipocytes, skeletal muscle cells, smooth muscle cells and the like.

Endodermal lineage cells are not particularly limited and may include, for example, gastrointestinal cells, pancreatic cells, liver cells, respiratory cells, and thyroid cells.

The "directionality control of differentiation induction of pluripotent stem cell" carried out using the control agent of the present invention refers to modifying the differentiation efficiency into cells of any lineage of the three germ layers (ectoderm, mesoderm, or endoderm) during differentiation induction of pluripotent stem cells as described above. As described below, the E-cadherin inhibitor contained in the control agent of the present invention has the function of alleviating intercellular adhesion mediated by E-cadherin. Therefore, by adding the control agent of the present invention to the culture system, the physical strength of intercellular adhesion in cell aggregates can be made "uniform," and by adjusting the amount of addition, the "uniform" strength of the intercellular adhesion can be controlled, thereby "controlling" the "directionality" of differentiation induction of a cell.

By making physical intercellular adhesion force uniform, the cell culture environment is also made uniform. This is hypothesized to reduce the number of cells deviating from the desired differentiated cell type when pluripotent stem cells are induced to differentiate. Furthermore, research by the inventors has shown that controlling physical intercellular adhesion allows for "directionality of differentiation induction" (it is at times referred to as "directional control of differentiation induction") towards any lineage of the three germ layers at the initial stages of differentiation induction. This is presumably because changing the amount of the control agent of the present invention in the differentiation induction system alters cell adhesion force, thereby affecting the intracellular environment, which in turn impacts the efficiency of differentiation into one of the desired lineages of the germ layers. Those of ordinary skill in the art can determine the appropriate amount of the control agent, treatment duration, and timing for adding the control agent to change the efficiency of differentiation into the desired cells.

### [E-cadherin inhibitor]

In the present specification, the E-cadherin inhibitor may be any substance capable of inhibiting intercellular adhesion caused by E-cadherin, and examples thereof include hemagglutinin, nucleic acids capable of inhibiting the expression of E-cadherin (e.g., antisense oligonucleotides, etc.), and antibodies against E-cadherin. Hemagglutinin is preferably used as the E-cadherin inhibitor.

### [Hemagglutinin (HA)]

In the present specification, the hemagglutinin is not particularly limited in terms of the method of acquisition, origin, and the like, as long as it can inhibit intercellular adhesion by E-cadherin. Examples thereof include hemagglutinin derived from Clostridium botulinum or bacteria related thereto. Clostridium botulinum may be any of type A, type B, type C, and type D. Hemagglutinin refers to a neurotoxin complex (also referred to as hemagglutinin complex) unless otherwise specified.

Hemagglutinin may be of a wild-type, a hemagglutinin variant, or a miniaturized hemagglutinin.

### [Hemagglutinin variant]

In the present specification, hemagglutinin may be a hemagglutinin variant to the extent that it has an effect of contributing to the directional control of differentiation induction of pluripotent stem cells. One or more embodiments of the hemagglutinin variant include, for example, a variant in which one, two, or three amino acid sequences of subcomponents have at least one amino acid mutation. Specific examples of the variant include the variant hemagglutinin (HA) complex protein described in WO2015/199243.

### [Miniaturized hemagglutinin]

In the present specification, moreover, hemagglutinin may be a miniaturized hemagglutinin to the extent that it has an effect of contributing to the directional control of differentiation induction of pluripotent stem cells. Examples of the miniaturized hemagglutinin include HA complex proteins that retain cadherin function inhibitory activity and lack all or part of the subcomponents. Specific examples of miniaturized hemagglutinin include the miniaturized hemagglutinin complex protein having an E-cadherin function inhibitory activity described in WO2019/103111.

In the present specification, unless particularly specified, "hemagglutinin" includes "hemagglutinin variant" and "miniaturized hemagglutinin". In addition, in the present specification, unless particularly specified, "hemagglutinin variant" and "miniaturized hemagglutinin" refer to those that have the effect of contributing to the directional control of differentiation induction of pluripotent stem cells.

The control agent of the present invention may also contain differentiation inducers suitable for the desired differentiated cells. Alternatively, the differentiation inducer may be provided as a separate reagent from the control agent and may be included in a kit with the control agent of the present invention. Using the control agent of the present invention in a differentiation system with a differentiation inducer suitable for the desired cells can modify, preferably improve, the differentiation efficiency into the desired differentiated cells.

By using the control agent of the present invention in the culture of pluripotent stem cells, directional control of differentiation induction of pluripotent stem cells is possible. The "directional control of differentiation induction of pluripotent stem cells" is as described above. Those of ordinary skill in the art can change the efficiency of differentiation induction into the desired cells by appropriately determining the desired differentiated cell type and the amount of the control agent of the present invention suitable for the differentiation induction system (differentiation inducer) used. For example, when using hemagglutinin as an E-cadherin inhibitor, the amount of the control agent suitable for the differentiation induction system used can be determined by varying the concentration of hemaggletinin between 1 and 80 nM as appropriate and testing the efficiency of differentiation induction into the desired cells. The amount of the control agent used in differentiation induction depends on the scale, culture conditions, and culture environment of the differentiation induction system to be applied, and it is not the kind of control agent that, if it used within this range, will induce into this cell type. It is understood that the control agent of the present invention is useful in the sense that those of ordinary skill in the art, based on their own experience, can alter the efficiency of differentiation induction by applying the control agent of the present invention to their own differentiation induction system.

Furthermore, the control agent of the present invention can be used to reduce or remove cells deviating from the desired differentiated cells in the differentiation induction system. The use of the control agent of the present invention leads to making uniform the physical intercellular adhesion strength in cell aggregates, which results in a uniform culture environment. This is hypothesized to reduce the number of cells deviating from the desired differentiated cells in the differentiation induction system. The term "Cells deviating from the desired differentiated cells" refer to cells that have been induced to be cells different from the desired differentiated cells or intermediate cells that have not been sufficiently induced to differentiate, and are mixed with the desired differentiated cells in a certain, unspecified differentiation induction system. Such deviating cells can be identified by cell morphology or expression of differentiation markers and should be removed in the production of cellular pharmaceutical. Techniques that can exclude these deviating cells during differentiation induction are highly valuable.

The culture of pluripotent stem cells is as described in the below-mentioned "2. The control method of the present invention".

### 2. The control method of the present invention

The present invention also provides a method for controlling the directionality of differentiation induction of pluripotent stem cells, including a step of culturing pluripotent stem cells in a medium containing an E-cadherin inhibitor. In the following, it is at times referred to as "the control method of the present invention".

In the control method of the present invention, the E-cadherin inhibitor, pluripotent stem cell, and directional control of differentiation induction of pluripotent stem cells are aided, as appropriate, by the description in "1. The control agent of the present invention".

The medium used for culturing pluripotent stem cells is not particularly limited as long as it supports the maintenance, proliferation, and/or differentiation induction of pluripotent stem cells. In one embodiment, the medium used for culturing pluripotent stem cells may contain an E-cadherin inhibitor and supports the maintenance of the undifferentiated state of pluripotent stem cells. Such a medium can be prepared by adding an E-cadherin inhibitor to a known pluripotent stem cell maintenance medium. In another embodiment, the medium used for culturing pluripotent stem cells may be a medium that contains an E-cadherin inhibitor and does not support the maintenance of the undifferentiated state of pluripotent stem cells. Such medium is not particularly limited as long as it contains an E-cadherin inhibitor and does not support the maintenance of the undifferentiated state of pluripotent stem cells. Examples thereof include media containing basal medium, serum and/or serum alternative, an E-cadherin inhibitor, preferably hemagglutinin (particularly hemagglutinin derived from Clostridium botulinum), and other components. It is preferable that the medium does not contain a substance that supports the maintenance of the undifferentiated state (e.g., bFGF, SCF, LIF, and the like), but this may not apply as long as the medium as a whole does not support the maintenance of the undifferentiated state of pluripotent stem cells.

As the basal medium, one type of synthetic medium used generally for culturing mammalian cells or multiple types thereof in combination can be used and, for example, commercial products such as Glasgow MEM (GMEM), IMDM (Iscove's Modified Dulbecco's medium), DMEM, NeuroBasal medium, NeuroBasal-A medium, F12-Ham, Kaighn's modified F12, and the like can be used.

As the serum, a serum derived from a mammal such as bovine, human, and the like can be used. The serum alternative is a low-protein replacement used instead of a serum such as FBS and the like used for cell culture. As commercially available products, for example, knockout Serum Replacement (KSR), Chemically-defined Lipid concentrated (manufactured by Gibco), Glutamax (manufactured by Gibco) and the like, and N2 and B27, which are serum alternatives for nerve cell culture, and the like are available. The concentration of serum or serum alternative can be appropriately set within the range of, for example, 0.5 - 30% (v/v). The concentration may be constant or stepwisely changed.

The concentration of the E-cadherin inhibitor is not particularly limited. Those of ordinary skill in the art can set the concentration appropriately in terms of reducing cells deviating from the desired differentiated cells in differentiation induction from pluripotent stem cells into any of the three germ layers, or in terms of modifying the efficiency of differentiation induction. For example, when using hemagglutinin as an E-cadherin inhibitor, the suitable concentration of hemagglutinin for differentiation induction system used can be determined by varying the concentration of hemagglutinin between 1 and 80 nM and testing the efficiency of differentiation induction into the desired cells.

Even if hemagglutinin is a variant of hemagglutinin, the concentration is not particularly limited for the same reasons as mentioned above. However, variants might be highly active in alleviating intercellular adhesion and therefore the reference concentration could be set at lower concentrations overall.

Even if hemagglutinin is miniaturized hemagglutinin, the concentration is also not particularly limited for the same reasons as mentioned above. However, miniaturized hemagglutinins generally tend to have lower activity in alleviating intercellular adhesion, so the reference concentration could be set at higher concentrations overall.

The medium can contain other components generally used for culture of mammalian cells, in addition to those mentioned above. Furthermore, a medium containing an E-cadherin inhibitor that does not support the maintenance of the undifferentiated state of pluripotent stem cells may also contain differentiation inducers suitable for the desired differentiated cells.

In the control method of the present invention, in order to realize uniform and homogeneous differentiation induction, the E-cadherin inhibitor may be added to (1) a medium for expansion culture while maintaining an undifferentiated state pluripotent stem cells, (2) a medium for differentiation induction from pluripotent stem cells into the desired specific differentiated cells, or (3) a medium (containing no differentiation inducer) that contains an E-cadherin inhibitor, does not induce differentiation into a specific differentiated cell, but does not support the maintenance of an undifferentiated state. In the case of (3), the cells obtained by the culture step can be further cultured in a medium (containing a differentiation inducer) for inducing differentiation into the desired specific, differentiated cells. The differentiation induction medium may or may not contain an E-cadherin inhibitor. Particularly, in the initial stages of differentiation induction (for example, in stages where specific differentiation such as embryoid body formation has not yet been induced), pluripotent stem cells can be contacted with the E-cadherin inhibitor. This allows for directionality towards any of the three germ layers, and subsequent cultivation with differentiation inducers can improve the uniformity, homogeneity, and yield of the desired differentiated cells. In other words, similar considerations apply to the "stabilizing method of the present invention" described later, where E-cadherin inhibitors can be applied.

The duration of culturing pluripotent stem cells in a medium containing an E-cadherin inhibitor can be set appropriately to the extent that the E-cadherin inhibitor achieves uniform intercellular adhesion strength and directionality of differentiation induction into the desired cells, and that prolonged contact with the E-cadherin inhibitor does not adversely affect the cells. For instance, when culturing pluripotent stem cells in a medium that does not support the maintenance of the undifferentiated state but supports differentiation, an E-cadherin inhibitor can be added shortly after seeding or 1 to several days post-seeding (e.g., 2, 3, 4, 5 days) and can continue to be added for more than one day (e.g., 1, 3, 5, 7, 10, 15, 20, 25, 30, 35, 40, 45, 50 days or more). Alternatively, for example, when pluripotent stem cells are cultured in the above medium (medium A) for about 3 to 7 days and then differentiated into desired differentiated cells using a differentiation inducing medium (medium B) containing a differentiation inducer, for example, immediately after seeding into medium A or one to several (e.g., 2, 3, 4, 5) days after seeding, the E-cadherin inhibitor is added and cultured, for example, for several hours to several days, preferably one to several (e.g., 5, 4, 3, 2) days, and then the cells can be further cultured in medium B which may or may not contain the E-cadherin inhibitor.

As the culture conditions for pluripotent stem cells in the control method of the present invention, a known method for inducing differentiation may be used. For example, an adherent culture method, a suspension culture method, and the like can be mentioned. As the adhesion culture method, a method using an adherent cell culture vessel, a method in which pluripotent stem cells are cultured in a state of being adhered to feeder cells (e.g., WO2001/088100), a method using a culture substrate coated with laminin E8 fragment (e.g., WO2015/053375), and the like are known, but the method is not limited thereto. The suspension culture method refers to a method of culturing a group of pluripotent stem cells that have gathered and formed a clump (floating aggregates) in a suspended state in a culture medium (e.g., WO2008/087917, Nature. 2011 Apr 7; 472(7341):51-6), but the method is not limited thereto.

### [Adhesion culture method]

The density of pluripotent stem cells to be used in culture is not particularly limited as long as pluripotent stem cells can be uniformly seeded, and adhesion culture is possible. It is desirably a density of the level permitting the intercellular adhesion to become uniform and appropriate control of the differentiation induction state, by the administration of an E-cadherin inhibitor when the cells are uniformly seeded and adhesion cultured. Such cell density can be appropriately determined. Other culture conditions such as culture temperature and CO₂ concentration can be set as appropriate. The culture period is not particularly limited as long as differentiation induction of pluripotent stem cells can be controlled. It is desirably a culture period that can maintain the effect of making intercellular adhesion uniform and can control differentiation induction by an E-cadherin inhibitor. This culture period can be appropriately changed according to the number of pluripotent stem cells used in the culture, culture equipment, culture substrate, culture method, and the like.

Culture substrates used in adhesion culture methods are not particularly limited and may include known substrates such as laminin E8 fragment-coated substrates.

### [Suspension culture method]

Cultures may also be performed using suspension culture methods in addition to adhesion culture. The number of pluripotent stem cells forming aggregates used for suspension culture should be sufficient to make the degree of intercellular adhesion uniform and the state of differentiation induction appropriately controlled by the administration of E-cadherin inhibitor. The number of cells can be set appropriately according to the desired differentiated cells and differentiation induction method to be applied. Other culture conditions such as culture temperature and CO₂ concentration can also be set as appropriate. The culture period is not particularly limited as long as differentiation induction of pluripotent stem cells can be controlled. It is desirably a culture period that can maintain the effect of making intercellular adhesion uniform and control differentiation induction by an E-cadherin inhibitor. This culture period can be appropriately changed according to the number of pluripotent stem cells used in the culture, culture equipment, culture substrate, culture method, and the like.

The cell culture vessel to be used in the suspension culture method is not particularly limited as long as it enables suspension culture of the cells. Examples of such cell culture vessel include flask, tissue culture flask, petri dish, tissue culture dish, multidish, microplate, microwell plate, micropore, multiplate, multiwell plate, chamber slide, tube, tray, culture bag, roller bottle and so on.

The differentiation inducers used in the control method of the present invention are not particularly limited and may include known inducers appropriate for the desired differentiated cells. Examples include Nodal signaling inhibitors, Wnt signaling inhibitors, Sonic Hedgehog signaling inhibitors, and Activin signaling enhancers, though these are merely illustrative and not limiting.

### 3. The stabilizing agent of the present invention

The present invention also provides an agent for stabilizing differentiation induction of pluripotent stem cells to a desired differentiated cell, containing an E-cadherin inhibitor. In the following, it is at times referred to as "the stabilizing agent of the present invention".

In the stabilizing agent of the present invention, the E-cadherin inhibitor, pluripotent stem cell, and directional control of differentiation induction of pluripotent stem cells are aided as appropriate, by the description in "1. The control agent of the present invention".

In the stabilizing agent of the present invention, the "differentiation induction" in the "stabilization of differentiation induction of pluripotent stem cells" is as described in "1. The control agent of the present invention". Furthermore, "stabilization of differentiation induction of pluripotent stem cells" using the stabilizing agent of the present invention refers to improving the efficiency of differentiation induction into the desired cells and/or achieving reproducible differentiation into the desired cells. By adding the stabilizing agent of the present invention to the culture system, the strength of physical intercellular adhesion in the cell aggregates can be made uniform, and the directionality of differentiation induction of cells is controlled, and in addition, by adjusting the amount of the agent, the strength of the uniformed intercellular adhesion can be adjusted to stabilize the differentiation induction into the desired differentiated cells.

In other words, by making the strength of physical intercellular adhesion uniform, the cell culture environment becomes uniform. This uniformity allows for improved efficiency of differentiation induction into any of desired lineage of the three germ layers and improved reproducibility, when pluripotent stem cells are induced to differentiate as described in the aforementioned "directional control of differentiation induction". Those of ordinary skill in the art can improve the differentiation efficiency and reproducibility into the desired differentiated cells by appropriately determining the amount of the stabilizing agent of the present invention. For instance, when using hemagglutinin as an E-cadherin inhibitor, the amount of the stabilizing agent of the present invention suitable for the differentiation induction system used can be determined by varying the concentration of hemaggletinin between 1 and 80 nM as appropriate and testing the efficiency of differentiation induction into the desired cells. The amount of the stabilizing agent used in differentiation induction depends on the scale, culture conditions, and culture environment of the differentiation system to be applied, and it is not the kind of stabilizing agent of the present invention that, if it used within this range, will induce into this cell type. It is understood that the stabilizing agent of the present invention is useful in the sense that those of ordinary skill in the art, based on their own experience, can alter the efficiency of differentiation induction by applying the stabilizing agent of the present invention to their own differentiation induction system.

Furthermore, the stabilizing agent of the present invention can be used to reduce or remove cells deviating from the desired cells in the differentiation system. The use of the stabilizing agent of the present invention leads to making uniform the physical intercellular adhesion strength in cell aggregates, which results in a uniform culture environment. This is hypothesized to reduce the number of cells deviating from the desired cells in the differentiation induction system. Furthermore, "Cells deviating from the desired cells" are as described above.

### 4. The stabilizing method of the present invention

The present invention also provides a method for efficiently producing the desired differentiated cells from pluripotent stem cells, in other words, a method for stabilizing differentiation induction into a desired cell. In the following, it is at times referred to as "the stabilizing method of the present invention". The method is not particularly limited as long as it includes a step of stabilizing differentiation induction into a desired cell by (1) a step of forming an aggregate of pluripotent stem cells, and (2) a step of culturing the aggregates obtained in step (1) in a medium comprising an E-cadherin inhibitor.

In the stabilizing method of the present invention, the pluripotent stem cell and E-cadherin inhibitor are aided as appropriate, by the description in "1. The control agent of the present invention".

### [Differentiation induction from pluripotent stem cells into desired cells]

The stabilizing method of the present invention is aimed at stabilizing the differentiation induction of pluripotent stem cells as starting cells into desired differentiated cells, thereby ultimately efficiently (more uniformly and homogeneously) producing a population of desired differentiated cells. It is preferred that pluripotent stem cells are in a state where intercellular adhesion exists, so the induction method of the present invention includes (1) a step of forming an aggregate of pluripotent stem cells.

In step (1), the "aggregate" refers to a clump formed by assembly of cells dispersed in a medium, in which the cells are adhered to each other. Therefore, colony, clump, cell clump, embryoid body, sphere, and spheroid are also encompassed in the aggregate. In cell culture systems, any state where cells adhere to each other to form a confluent layer is also considered as an aggregate. Aggregates of pluripotent stem cells are formed, for example, by achieving a confluent state through adhesion culture methods and also by culturing pluripotent stem cells using a suspension culture method. Examples of such medium and culture method are the same as those described above.

As the medium and culture method used in step (2), those similar to the ones described in "2. The control method of the present invention" can be mentioned. Thus, as in the view point described in "2. The control method of the present invention", the stabilizing method of the present invention can also stabilize differentiation induction into desired differentiated cells by using E-cadherin inhibitors in each step of the differentiation induction into the desired differentiated cells.

The desired differentiated cells may include, for example, ectodermal lineage cell, mesodermal lineage cell, and endodermal lineage cell.

Ectodermal lineage cells are not particularly limited but may include, for example, neuronal cells and epidermal cells. Specifically, retinal pigment epithelial cells are mentioned as examples.

Mesodermal lineage cells are not particularly limited and may include, for example, hematopoietic cells (including blood-lineage cells), vascular system cell (vascular endothelial cells etc.), cardiomyocytes (e.g., atrial myocytes, ventricular myocytes etc.), osteocytes, chondrocytes, tendon cells, adipocytes, skeletal muscle cells, smooth muscle cells, and the like.

Endodermal lineage cells are not particularly limited and may include, for example, gastrointestinal cells, pancreatic cells, liver cells, respiratory cells, and thyroid cells.

The concentration of the E-cadherin inhibitor is not specifically limited. Those of ordinary skill in the art can set the concentration appropriately in terms of reducing cells deviating from the desired differentiated cells in differentiation induction from pluripotent stem cells into any of the three germ layers, or in terms of enhancing the efficiency of differentiation induction and reproducibility, in other words, in terms of stabilizing differentiation induction into the desired differentiated cells. For example, when using hemagglutinin as an E-cadherin inhibitor, the suitable hemagglutinin concentration for differentiation induction system used can be determined by varying the concentration of hemagglutinin between 1 and 80 nM and testing the efficiency of differentiation induction into the desired cells.

Even if hemagglutinin is a variant of hemagglutinin, the concentration is not particularly limited for the same reasons as mentioned above. However, variants may be highly active in alleviating intercellular adhesion and therefore the reference concentration could be set at lower concentrations overall.

For miniaturized hemagglutinin, the concentration is also not specifically limited for the same reasons as mentioned above. However, miniaturized hemagglutinins generally tend to have lower activity in alleviating intercellular adhesion, and therefore the reference concentration could be set at higher concentrations overall.

### [Example]

The present disclosure is described in more detail below with reference to the following examples, but they are merely illustrative and the present disclosure is not limited to these examples.

### [Example 1] directionality of differentiation induction using hemagglutinin

In order to examine whether directionality of differentiation induction can be achieved by using hemagglutinin, a basic medium for differentiation induction into retinal pigment epithelial cells (RPE cells) was used without using differentiation inducers (e.g., SB431542 and CKI-7, etc.) that promote the differentiation induction of retinal pigment epithelial cells. In this case, the effect of adding Clostridium botulinum-derived hemagglutinin at various concentrations on the differentiation induction was observed. The specific procedure is as follows.

The reagents used were as follows.
· differentiation induction basic medium (Glasgow's MEM (GMEM) medium (Invitrogen), KnockOut^{™}Serum Replacement (KSR) (Invitrogen), 0.1 mM MEM non-essential amino acid solution (Invitrogen), 1 mM sodium pyruvate (SIGMA), StemSure (registered trademark) 10 mmol/l 2-mercaptoethanol (×100) (Wako Pure Chemical Industries, Ltd.), 100 U/ml penicillin-100 µg/ml streptomycin (Invitrogen))
· first differentiation induction medium (differentiation induction basic medium containing 20% KSR, 10 µM Y-27632 (Wako Pure Chemical Industries, Ltd.), a given concentration of hemagglutinin (Table 1))
· second differentiation induction medium (differentiation induction basic medium containing 15% KSR, 10 µM Y-27632 (Wako Pure Chemical Industries, Ltd.), a given concentration of hemagglutinin (Table 1))
· third differentiation induction medium (differentiation induction basic medium containing 10% KSR, 10 µM Y-27632 (Wako Pure Chemical Industries, Ltd.), a given concentration of hemagglutinin (Table 1))

· fourth differentiation induction medium (differentiation induction basic medium containing 10% KSR, a given concentration of hemagglutinin (Table 1))
· RPE maintenance medium (67% DMEM.low glucose (SIGMA), 29% F12 (SIGMA), 1.9 mM L-glutamine (Invitrogen), 1.9% B-27 supplement (Invitrogen), 96 U/mL penicillin sodium, 96 µg/mL Streptomycin sulfate)

Human skin-derived iPS cells (253G1, provided by Kyoto University) were seeded onto a laminin-coated culture dish (manufactured by Sumika Bakelite Co., Ltd.) at 9×10⁶ cells/9 cm dish. The laminin-coated culture dish was prepared by coating 9 cm culture dish (BD FALCON) with a 0.5 µg/cm² solution of laminin 511E8 fragment (a protein disclosed in Example (3) of WO2011043405, provided by Osaka University) at 37 degrees for 1 hr or longer. The iPS cells quickly adhered to the culture dish, and formation of floating aggregates was not observed.

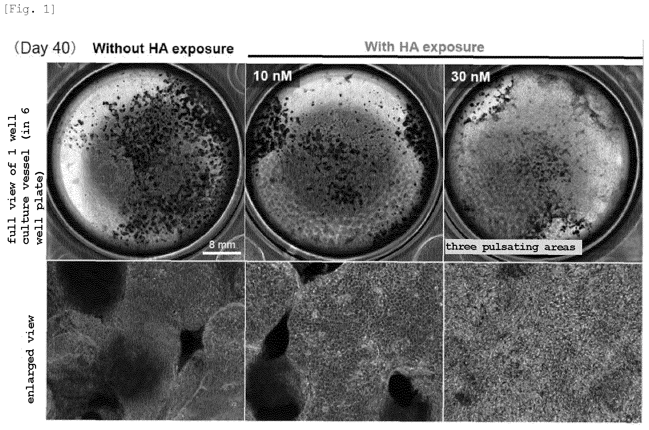

The first day of culture was set as Day 0, and the entire medium was exchanged every day from the start of culture (Day 1) until around Day 40, when pigment cells were confirmed. The composition of the medium was changed stepwise as shown below: differentiation induction basic medium was used on Day 0, the first differentiation induction medium (20% KSR) on Days 1-4, the second differentiation induction medium (15% KSR) on Days 5-8, the third differentiation induction medium (10% KSR) on Days 9-12, and the fourth differentiation induction medium (10% KSR) from Day 13 until around Day 40. The effect of hemagglutinin, added at different concentrations to each medium, on differentiation induction was observed. The experiment conditions (conditions 1-3) are shown in Table 1 and the results are shown in Table 1 and Fig. 1.

**[Table 1]**

| Test conditions | condition 1 | condition 2 | condition 3 |
|---|---|---|---|
| medium | differentiation induction basic medium | | |
| w/wo HA complex addition | wo HA complex addition | w 10 nM HA complex addition | w 30 nM HA complex addition |
| formation of cell aggregate in three-dimensional layered structure | cell aggregate is large | cell aggregate becomes smaller | aggregate is not formed |
| differentiation into RPE cell (formation of cell aggregate with dark pigment) | formation of dark pigment found in some large cell aggregate | formation of dark pigment found throughout small cell aggregate | none |
| differentiation into other cell (proportion in culture vessel) | retinal pigment epithelial cell <hepatocyte-like cell | retinal pigment epithelial cell <<hepatocyte-like cell | hepatocyte-like cell <<<muscle cell (some cardiac muscle cell) |

| | | | |
|---|---|---|---|
| HA: hemagglutinin | | | |

When differentiation induction was performed without adding hemagglutinin, retinal pigment epithelial cells, which are cells with dark pigment, were gradually induced from the cell aggregates of pluripotent stem cells (Table 1 (condition 1), left column in Fig. 1). On the other hand, by adding hemagglutinin to the culture system to weaken the strength of intercellular adhesion in cell aggregates, hepatocyte-like cells were induced at a concentration of 10 nM (Table 1 (condition 2), center column in Fig. 1), and muscle cells and cardiomyocytes were induced at a concentration of 30 nM (Table 1 (condition 3), right column in Fig. 1).

### [Example 2] Examination of direct differentiation of pluripotent stem cells using hemagglutinin

Next, cell aggregates of pluripotent stem cells were treated with hemagglutinin at varying concentrations for 24 hours. Subsequently, differentiation into each of the three germ lineages was induced using the STEMdiff Trilineage Differentiation Kit (STEMCELL Technologies) and the differentiation potential was assessed by RT-PCR.

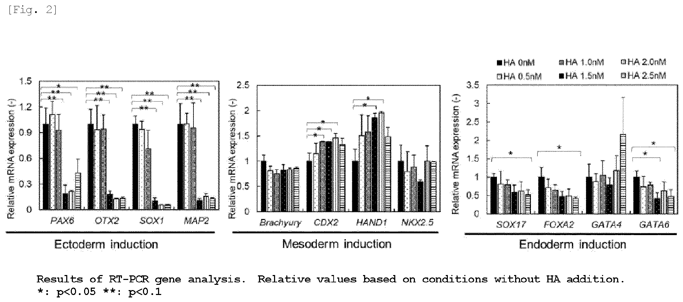

Human iPS cells, 1383D2, were seeded into Elplasia roundbottom plates (Corning) with 200 cells/dimple and cultured for 1 day in StemFit AK02N medium containing ROCK inhibitor. Following this, the cells were cultured for 5 days in StemFit AK02N medium without ROCK inhibitor. They were then cultured for 24 hours in StemFit AK02N medium containing hemagglutinin at varying concentrations. After this, the cells were differentiated into each germ layer using the STEMdiff Trilineage Differentiation Kit, and gene analyzation was performed by RT-PCR after 6 days. The results are shown in Fig. 2.

In the differentiation induction into ectodermal lineage, when the hemagglutinin concentration was low (0 nM to 1 nM), the expression levels of ectodermal markers such as PAX6, OTX2, SOX1, and MAP2 were significantly increased. On the other hand, in the differentiation induction into mesodermal lineage, the expression levels of mesodermal markers such as CDX2 and HAND1 were significantly increased at intermediate hemagglutinin concentrations (1 nM to 2 nM). Furthermore, in the differentiation induction into endoderm lineage, when the hemagglutinin concentration was high (2.5 nM), the expression levels of endodermal markers such as SOX17, FOXA2, and GATA6 were significantly reduced, suggesting that lower hemagglutinin concentrations (0 nM to 1 nM) are more likely to induce differentiation into the endoderm lineage.

The above results clarified that the direction of differentiation induction into any of the three germ layers can be controlled by adjusting the concentration of hemagglutinin added.

### [Example 3] Stabilization of cardiomyocyte differentiation of pluripotent stem cells using hemagglutinin

### (1) Test of hemagglutinin treatment before differentiation induction (test of culture system suitable for mass culture)

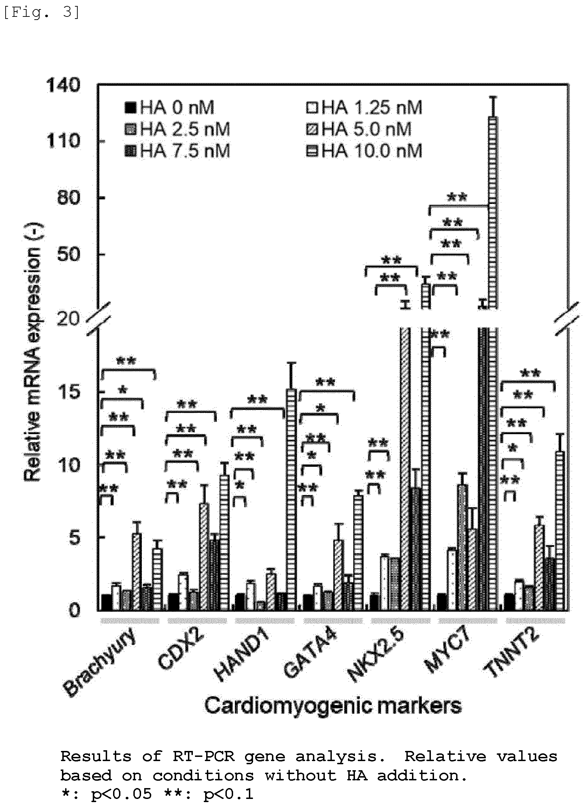

Human iPS cells, 1383D2, were seeded into Elplasia roundbottom plates with 200 cells/dimple and cultured for 1 day in a medium containing ROCK inhibitor. Following this, the cells were cultured for 2 days in StemFit AK02N medium without ROCK inhibitor. They were then cultured for 24 hours in StemFit AK02N medium containing hemagglutinin at varying concentrations. Thereafter, the cells were cultured for 14 days in a known medium for inducing differentiation into cardiomyocytes to induce differentiation into cardiomyocytes. Differentiation induction was performed with reference to the description in Sougawa et al., Methods Mol Biol. 2320: 23-27 (2021). Genetic analysis of cardiomyocyte markers was performed by RT-PCR 18 days after seeding of the iPS cells. The results are shown in Fig. 3.
· first differentiation induction medium (Liquid C-free StemFit AK02N medium, Y-27632, BMP-4)
· second differentiation induction medium (Liquid C-free StemFit AK02N medium, BMP-4, ActivinA, bFGF)
· third differentiation induction medium (Liquid C-free StemFit AK02N medium, IWP-3, SB431542, Dorsomorphin, VEGF) · fourth differentiation induction medium (Liquid C-free StemFit AK02N medium, bFGF, VEGF)

It was found that the expression level of genes specific to cardiomyocytes increased along with an increase in the hemagglutinin concentration. This suggests that differentiation can be stably induced even in cell aggregates and that the addition of hemagglutinin can stabilize the differentiation induction step even in large-scale culture systems.

### (2) Test to induce differentiation by adding hemagglutinin

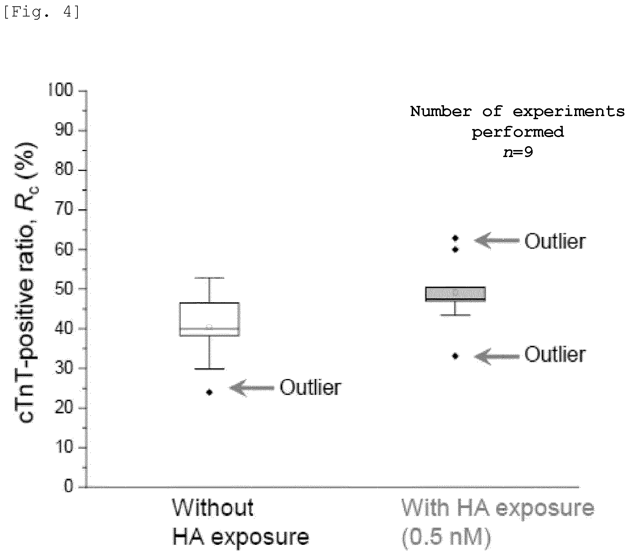

The test was performed in the same manner as in the above-mentioned (1), except that hemagglutinin was added to a known medium for differentiation induction into cardiomyocytes, and no treatment with hemagglutinin was performed before inducing differentiation into cardiomyocytes. The proportion of cells expressing cTnT, which is a gene specific to cardiomyocytes, was measured (n=9), and the results thereof are shown in a box-and-hide diagram (Fig. 4).

When the cardiac differentiation induction marker cTnT was used as an indicator, the differentiation induction system with added hemagglutinin showed a tendency toward an increase in the proportion of cTnT marker positive cells, as compared with conventional differentiation induction without addition of hemagglutinin, and the variance also became smaller. In other words, it was suggested that by introducing a hemagglutinin treatment step into a system for inducing differentiation into cardiomyocytes, a population of cardiomyocytes can be efficiently obtained and that this has the action of reducing the inconsistency in the results between cultures (potentially stabilizing the differentiation induction into cardiomyocytes).

### [Example 4] Stabilization of hepatocyte differentiation of pluripotent stem cells using hemagglutinin (Test of culture system suitable for large-scale culture)

Human iPS cells, 1383D2, were seeded into Elplasia roundbottom plates with 200 cells/dimple and cultured for 1 day in a medium containing ROCK inhibitor. Following this, the cells were cultured for 1 day in StemFit AK02N medium without ROCK inhibitor. They were then cultured for 3 days in StemFit AK02N medium containing hemagglutinin at varying concentrations of 0 nM, 1 nM, 5 nM, and 10 nM. The medium was then washed, and differentiation induction into hepatocytes was started. Differentiation induction was performed by reference to the description in Kajiwara et al., Proc Natl Acad Sci USA. 109(31): 12538-43. Specifically, the cells were first cultured for 3 days in RPMI1640 medium (Nacalai) containing 1 × B27 supplement (Gibco), 100 ng/mL activin A (R&D systems), 50 ng/mL Wnt3a (R&D systems) and 0.5 mM NaB (Sigma) to induce endoderm. After washing the medium, the cells were then cultured for 7 days in Knockout-DMEM (Gibco) containing 20% (v/v) KSR (Gibco), 1 mM L-glutamine, 1%. (v/v) NEAA, 0.1 mM 2-mercaptoethanol and 1% (v/v) DMSO to induce hepatocytes. After washing the medium, the cells were finally cultured for 7 days in Hepatocyte Culture Medium BulletKit (manufactured by Lonza) containing 20 ng/mL HGF (PeproTech) and 20 ng/mL Oncostatin M (PeproTech) to mature hepatocytes.

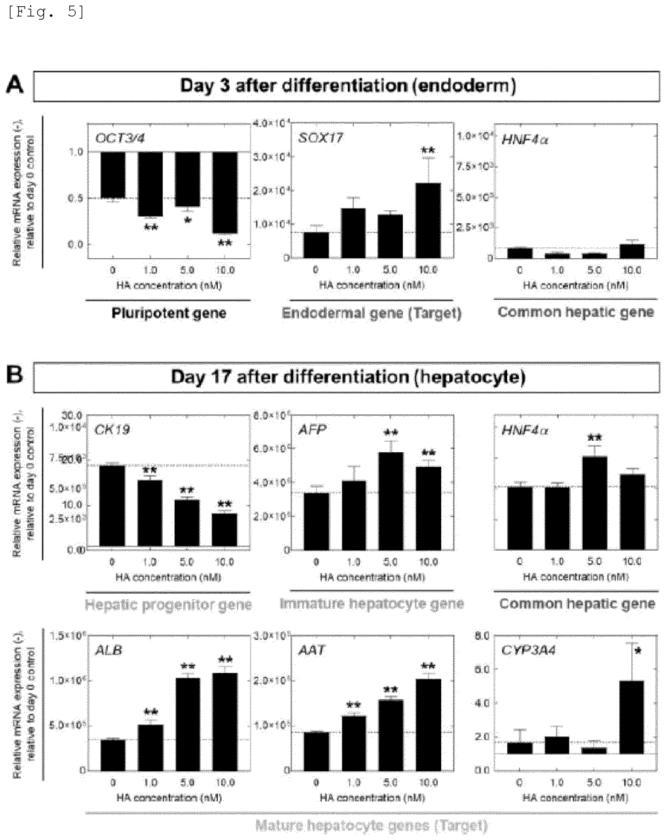

Genetic analysis of hepatocyte markers was performed by RT-PCR 3 days after hepatocyte induction (after endoderm induction) and further, 17 days later (after hepatocyte maturation). The results are shown in Fig. 5.

After 3 days (A), it was found that as the hemagglutinin concentration was increased, the expression level of Oct3/4 decreased, while the expression level of an endoderm-specific gene (Sox17) increased. Furthermore, after 17 days (B), it was found that as the hemagglutinin concentration was increased, the expression level of a hepatic progenitor cell marker (CK19) decreased, while the expression level of a group of genes (ALB, AAT, CYP34) specific to mature hepatocytes was increased. In other words, it was suggested that by introducing a hemagglutinin treatment step into a system for inducing differentiation into hepatocytes, a population of hepatocytes can be efficiently obtained (potentially stabilizing the induction of differentiation into hepatocytes).

From the above, it was shown that the effect of loosening intercellular adhesion by adding hemagglutinin has the effect of changing the induction of differentiation into retinal pigment epithelial cells into hepatocytes or muscular cells, and further has the effect of stabilizing the induction step of cardiomyocytes and hepatocytes, and efficiently obtaining cardiomyocytes and hepatocytes. Therefore, when inducing the differentiation of pluripotent stem cells into desired cells under any differentiation induction conditions, addition of an appropriate concentration of hemagglutinin can efficiently induce differentiation into the desired cells.

### [Industrial Applicability]

The method and composition according to the present disclosure are useful in, for example, the field of regenerative medicine.

This application is based on a patent application No. 2022-017548 filed in Japan (filing date: February 7, 2022), the contents of which are incorporated in full herein.

## Claims

1. An agent for directional control of differentiation induction of a pluripotent stem cell, comprising an E-cadherin inhibitor.

2. The agent according to claim 1, wherein the pluripotent stem cell is an embryonic stem (ES) cell or an induced pluripotent stem (iPS) cell.

3. The agent according to claim 1 or 2, wherein the directional control of differentiation induction of pluripotent stem cell is a change in the differentiation efficiency of pluripotent stem cell into any of the three germ lineages.

4. The agent according to claim 1 or 2, wherein the directional control of differentiation induction of pluripotent stem cell is a reduction or removal of cells that deviatefrom the desired differentiated cell.

5. The agent according to claim 1 or 2, wherein the E-cadherin inhibitor is hemagglutinin.

6. A method for controlling the directionality of differentiation induction of a pluripotent stem cell, comprising a step of culturing a pluripotent stem cell in a medium comprising an E-cadherin inhibitor.

7. The method according to claim 6, wherein the culture is performed in the presence of a differentiation inducer.

8. The method according to claim 6 or 7, wherein, in the step of culturing a pluripotent stem cell in a medium comprising an E-cadherin inhibitor, a concentration of the inhibitor is adjusted.

9. The method according to claim 6 or 7, wherein the directional control of differentiation induction of pluripotent stem cell is a change in the differentiation efficiency of pluripotent stem cell into any of the three germ lineages.

10. The method according to claim 6 or 7, wherein the E-cadherin inhibitor is hemagglutinin.

11. An agent for stabilizing differentiation induction of a pluripotent stem cell, comprising an E-cadherin inhibitor.

12. The agent according to claim 11, wherein the stabilization of differentiation induction of pluripotent stem cell is an improvement in the differentiation efficiency of pluripotent stem cell into any of the three germ lineages.

13. The agent according to claim 11, wherein the stabilization of differentiation induction of pluripotent stem cell is a reduction or removal of cells that deviate from the desired differentiated cell.

14. The agent according to any one of claims 11 to 13, wherein the E-cadherin inhibitor is hemagglutinin.

15. A method for producing a desired differentiated cell from a pluripotent stem cell, comprising the following steps:
(1) a step of forming an aggregate of pluripotent stem cells, and
(2) a step of culturing the aggregates obtained in step (1) in a medium comprising an E-cadherin inhibitor, thereby to stabilize the differentiation induction into the desired differentiated cell.

16. The method according to claim 15, wherein the culture is performed in the presence of a differentiation inducer.

17. The method according to claim 15 or 16, wherein the pluripotent stem cell is an ES cell or an iPS cell.

18. The method according to claim 15 or 16, wherein the E-cadherin inhibitor is hemagglutinin.
